# EUROPEAN PATENT SPECIFICATION

(11) **EP 2 805 164 B1**
(45) Date of publication and mention of the grant of the patent: **08.11.2017**
(21) Application number: 13707917.4
(22) Date of filing: 14.01.2013
(51) Int. Cl.: G01N 33/50, G01N 33/543, G01N 33/58

(54) **DETERMINING A PRESENCE OF TARGET MOLECULES IN A BODY FLUID COMPRISING CELLS**
NACHWEIS DER ANWESENHEIT EINES ZIELMOLEKÜLS IN EINER ZELLENENTHALTENDEN KÖRPERFLÜSSIGKEIT
DÉTERMINATION DE LA PRÉSENCE D'UNE MOLECULE CIBLE DANS UN FLUIDE CORPOREL COMPRÉNANT DES CELLULES

(30) Priority: 16.01.2012 US 201261586879 P
(43) Date of publication of application: 26.11.2014
(73) Proprietor: Koninklijke Philips N.V., 5656 AE Eindhoven (NL)
(72) Inventor: DE KIEVIT, Peggy, NL-5656 AE Eindhoven (NL); DE REGT, Bram, NL-5656 AE Eindhoven (NL); DITTMER, Wendy Uyen, NL-5656 AE Eindhoven (NL); ORSEL, Joukje Garrelina, NL-5656 AE Eindhoven (NL)
(74) Representative: Steffen, Thomas
(86) International application number: PCT/IB2013/050335
(87) International publication number: WO 2013/108168

(56) References cited:
- WO-A1-2010/073182
- WO-A2-2011/069029
- US-A1- 2009 291 508
- US-A1- 2011 059 556
- US-A1- 2011 136 258
- MANI VIGNESHWARAN ET AL: "Ultrasensitive immunosensor for cancer biomarker proteins using gold nanoparticle film electrodes and multienzyme-particle amplification", ACS NANO, AMERICAN CHEMICAL SOCIETY, US, vol. 3, no. 3, 24 March 2009 (2009-03-24), pages 585-594, XP009148610, ISSN: 1936-086X, DOI: 10.1021/NN800863W [retrieved on 2009-02-13]
- ELHAM SHARIF ET AL: "Novel immunoassay technique for rapid measurement of intracellular proteins using paramagnetic particles", JOURNAL OF IMMUNOLOGICAL METHODS, vol. 388, no. 1-2, 14 December 2012 (2012-12-14), pages 78-85, XP055059265, ISSN: 0022-1759, DOI: 10.1016/j.jim.2012.11.015

## Description

### FIELD OF THE INVENTION

The invention relates to a method of determining a presence of target molecules in a body fluid comprising cells, and a cartridge for the analyzer device.

### BACKGROUND OF THE INVENTION

The detection of target molecules in a body fluid like blood may be a challenge for many detection techniques. In particular, optical techniques such as fluorescence and colorimetry may have the disadvantage that red blood cells result in a high optical background (e.g. autofluorescence, high absorption). Near field optical techniques like the FTIR (frustrated total internal reflection) technique may be less sensitive to these types of optical interferences. Together with the FTIR technique magnetic particles may be used for detecting target molecules bound to a substrate surface, wherein the magnetic particles may be guided to the substrate with magnetic fields. However when using magnetic particles, they may have a tendency to irreversibly attach to the blood cells and red blood cells may interfere with the magnetic actuation process as they occupy space on the substrate surface and may exclude magnetic particles from binding.

The most common method to remove blood cells is to remove them via a centrifugal force or a separation technique (e.g. filter, direct capture). The result is a clear blood plasma matrix which may be easier to do analysis on.

The highest quality plasma may be obtained by centrifuging blood. This however in most cases requires the use of a motor with moving parts. In order to avoid moving parts, high power consumption and expensive parts, most point of care analyzer devices employ a filter as an inexpensive and simple solution. Using a filter may have the disadvantage that it may have a high surface area on which adsorption (so called retention) of target molecules is often observed. Thus for each type of target molecules to be detected, chemical measures may have to be taken to block the filter, usually with a protein, so that the target molecules cannot bind to the filter. However, for each new type of target molecule to be detected a blocking molecule that does not interfere with the assay may have to be found, which may not be possible in all cases and in some cases a small amount of retention may be observed. For high sensitivity and high precision analytical determinations the use of a filter may not be appropriate for the above reasons.

### SUMMARY OF THE INVENTION

It may be an object of the invention to provide a simple, cheap and accurate method of detecting target molecules that may be performed at the point of care. In particular, an object of the invention is to provide such a method that may be performed by a point of care and/or handheld analyzer device to achieve a performance observed in central lab analyzers, which use centrifugation techniques.

This object is achieved by the subject-matter of the independent claims. Further exemplary embodiments are evident from the dependent claims and the following description.

An aspect of the invention relates to a method of determining a presence or an amount of target molecules in a body fluid comprising cells, for example in blood.

According to the invention, the method comprises the steps of: lysing cells in the body fluid for generating a lysate; mixing the lysate with particles with first specific binding molecules; binding target molecules to the particles through first specific binding molecules; exposing a substrate with second specific binding molecules to the lysate; binding target molecules to the substrate through second specific binding molecules; detecting particles attached to the substrate.

The method may be used for the direct detection of biological target molecules, in particular proteins, in blood. It may be seen as a gist of the invention that a sample of the body fluid is first lysed and thereafter the lysate may be used directly to determine the target molecule concentration, for example, in a magnetic label assay with the magnetic particles and the substrate. In particular, the analytical determination of the target molecules may be performed directly in the lysate (in particular without removing any constituents of the body fluid or the lysate).

The target molecule concentration may be determined with an optical detection technique, for example a FTIR technique, in which the target molecule concentration is determined from light reflected from the substrate with bound magnetic particles. The FTIR technique and/or the magnetic label technique may be performed directly in a full blood sample such that interference from cells is not present.

Further aspects of the disclosure relate to an analyzer device with which the method as described in the above and the following may be performed. The invention also relates to a disposable cartridge for insertion into an analyzer device.

It has to be understood that features of the analyzer device and/or the cartridge as described in the above and in the following may be features of the method as described in the above and in the following and vice versa.

According to the invention, the cartridge comprises a detergent for lysing cells in a body fluid, particles with first specific binding molecules and a substrate with second specific binding molecules. A sample of body fluid may be put into the cartridge, lysed by the detergent and may be analyzed in the analyzer device as described in the above and in the following.

These and other aspects of the invention will be apparent from and elucidated with reference to the embodiments described hereinafter.

### BRIEF DESCRIPTION OF THE DRAWINGS

Below, embodiments of the present invention are described in more detail with reference to the attached drawings.
Fig 1 schematically shows an analyzer device according to an embodiment of the invention.
Fig. 2 schematically shows a cartridge according to an embodiment of the invention.
Fig. 3 schematically shows a detail view of the analyzer device with the cartridge inserted according to an embodiment of the invention.
Fig. 4 shows a flow diagram for a method for detecting target molecules according to an embodiment of the invention.
Fig. 5 shows a diagram with detection signal changes caused by lysing a blood sample without target molecules according to an embodiment of the invention.
Fig. 6 shows a diagram with detection signal changes caused by lysing a blood sample with target molecules according to an embodiment of the invention.

In principle, identical parts are provided with the same reference symbols in the figures.

### DETAILED DESCRIPTION OF EMBODIMENTS

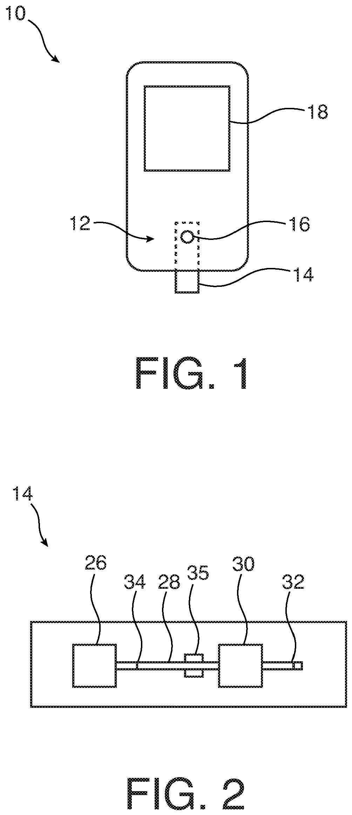

Fig. 1 schematically shows a handheld analyzer device 10 that may be used at a point of care, for example a physician's office, a hospital bedside, a ambulance and a patient's home. The handheld analyzer device may be used for rapid medical diagnosis outside of laboratory environments.

The handheld analyzer device has a slot 12 for receiving a disposable cartridge 14. A sample of body fluid 16 like blood, which has been taken from a patient, may be put onto the cartridge 14. The cartridge 14 may be inserted into the slot 12 in the analyzer device 10. The sample 16 may be analyzed by the analyzer device 10 and the result may be directly displayed on a display 18 of the analyzer device 10.

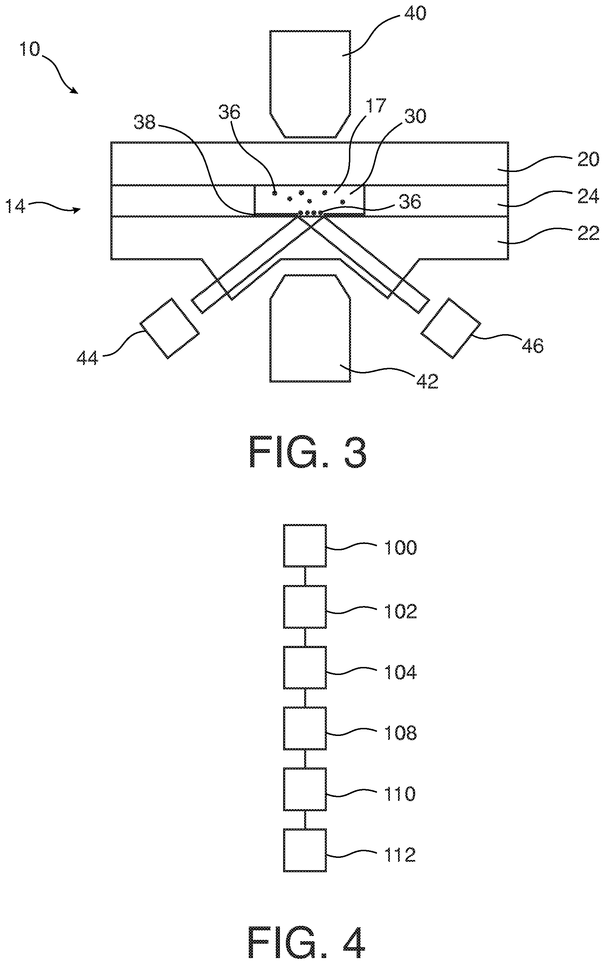

Fig. 2 schematically shows the cartridge 14. The cartridge 14 comprises a first cavity or inlet 26 for receiving a sample of body fluid 16, a capillary transportation channel 28, a second cavity or sample chamber 30 for analyzing body fluid 16 and a vent 32. With respect to Fig. 3, the cartridge 14 may comprise a top part 20 and a bottom part 22, which may be manufactured from injection-molded plastic. The two parts 20, 22 may be attached together with an adhesive tape 24 to form the cartridge 14.

The first cavity 26 may be formed as an inlet 26 in which lysis chemicals (a detergent) may be deposited.

According to the invention, the cartridge 14 comprises a detergent for lysing cells in a body fluid 16, in particular in the first cavity 26.

According to the invention, the cartridge comprises a first cavity 26 and a second cavity 30 connected by a channel 28.

According to the invention, the detergent is arranged in the first cavity 26.

According to the invention, the cartridge comprises a valve 34 in the channel 28. The channel 28 comprises a valve 34 that opens after a specific time after the sample of body fluid 16 has been deposited onto the inlet 26. For example, the valve 34 may be magnetically, electrically and/or fluidically actuated to release.

According to the invention, the valve 34 is adapted to open after a specific time after the body fluid 16 has entered the first cavity 26.

The channel 28 may comprise a mixing structure 35, for example a static microfluidic structure, a mixing structure driven by mechanical, magnetic, electrical and/or optical forces. The mixing structure 35 may allow the lysing agent to rapidly distribute in the blood for quick lysis. This may remove the need for a valve 34 or only a valve at the end of the channel that is closed shortly. The mixing structure 35 may also be contained in the first cavity 26, in the channel 28, and/or in the cavity 30 with the assay if lysis is done simultaneous to the assay.

Fig. 3 shows schematically shows a cross-sectional view through the second cavity 30 of the cartridge 14 inserted into the analyzer device 10. In the second cavity 30, magnetic particles 36 are arranged, which comprise first specific binding molecules (also called thereafter "first detector molecules") that may be adapted to bind to a specific type of target molecule. It has to be noted that magnetic particles 36 may be any type of particles that react to magnetic fields, for example that may be moved with the aid of magnetic fields. The particles 36 may be nanoparticles.

The second cavity 30 comprises a substrate surface 38, which comprises second specific binding (also called thereafter "second detector molecules") that may also be adapted to bind to the specific type of target molecule.

The first detector molecules and the second detector molecules may be of the same type or may be of different types.

Detector molecules may be any type of specific binding species, for example, antibodies, aptamers, anti-sense nucleic acids and/or antibody fragments etc.

According to the invention, the cartridge 14 comprises particles 36, for example magnetic particles 36, with first detector molecules and a substrate 38 with second detector molecules.

According to the invention, the particles 36 and the substrate 38 are arranged in the second cavity 30.

When lysed body fluid 17 or lysate 17 from the first cavity 26 enters the second cavity 30 via the channel 28, target molecules in the lysed body fluid 17 bind thought the first detector molecules to the magnet particles 36 and through the second detector molecules to the substrate surface 38. In such a way, magnetic particles 36 are bound to the substrate surface 38. The amount of magnetic particles 36 bound to the substrate surface 38 depends on the amount of target molecules in the body fluid 16.

For supporting the binding reaction to the substrate surface 38, the analyzer device 10 comprises two controllable electromagnets 40, 42 for moving the magnetic particles 36 towards the substrate surface 38 and away from the substrate surface. The two magnets 40, 42 are placed respectively above and below the cartridge 14 and in particular the second cavity 30.

For detecting the magnetic particles 36, the analyzer device 10 has a light source 44, for example a LED, and a light detector 46, for example a CCD camera. The light source 42 radiates a beam of light through the bottom part 22 of the cartridge onto the substrate surface 38. The beam of light is at least partially reflected and falls onto the light detector 46. From the degree of reflection, the amount of magnetic particles 36 bound to the substrate surface and thus indirectly the amount of target molecules in the body fluid 16 may be determined. Without magnetic particles 36 bound to the substrate surface 38, the light is totally reflected at the boundary between the body fluid 16 and the bottom part 22 of the cartridge 14. Furthermore, an evanescent field decaying exponentially and penetrating the lyzed body fluid 17 only a subwavelength distance is created. When magnetic particles 38 are present, they absorb and scatter a part of the evanescent field, and disturb the total reflection of the light, theryby reducing the intensity of the reflected light. This technique may be called frustrated total internal reflection (FTIR).

Fig. 4 shows a flow diagram for a method for detecting target molecules in a body fluid, which will be explained with respect to the Fig. 1 to 3.

For example the body fluid 16 may be full blood, i.e. blood without any constituents removed.

According to an embodiment of the invention, the body fluid 16 comprises or is blood.

According to an embodiment of the invention, the target molecules are a specific type of protein.

According to an embodiment of the invention, the target molecules are at least one of: cardiac troponin I (cTnI), cardiac troponin T, natriuretic peptides (BNP, NT-proBNP), myoglobin, creatine kinase MB, d-dimer, procalcitonin and parathyroid hormone.

In step 100, a sample of body fluid 16 is provided and deposited at the inlet 26, first chamber or first cavity 26 of the cartridge. The cartridge 14 may be inserted into the slot 12 of the analyzer device 10 directly after the depositing of the body fluid 16 in the cartridge 14.

In step 102, the body fluid 16 is lysed. For example, a detergent in the first cavity 26 may lyse the cells in the body fluid 16.

According to the invention, the method comprises lysing cells in the body fluid 16 for generating a lysate 17. Lysis may be defined as a process in which cell are destroyed. It may be possible that the cells lyse before the assay is performed in the resulting lysate. However, the lysis and the assay may be performed simultaneously.

According to the invention, the cells are lysed by a detergent. A detergent may be any substance adapted for lysing cells.

Lysis chemicals (detergent) may be immobilized on various surfaces of the cartridge 14 (eg. inorganic (eg. glass) and organic (eg. plastics)) using a method as adsorption. The detergents may be present in a dry form, for example with an anticoagulant (throughout the entire cartridge or at the first cavity 26). The detergent may be present with the magnetic particles 36 which also may be present in a dry form. The detergents may be immobilized in the microfluidic structure of the cartridge 14 such that the body fluid 16 may be directly exposed to the detergent quickly after sample deposition.

According to an embodiment of the invention, the detergent comprises at least one of Nonidet-P40, Nonidet-P40 substitute, sucrose monolaurate, Chemal LA-9 and Triton X100. These detergents may be highly effective to lyse red blood cells. In particular, only little interference with an cTnI assay is observed.

Alternatively or additionally, according to an embodiment of the invention, the cells are lysed by a physical lysing method. For example, the cells may be lysed by sonication, i.e. by exposing them to ultrasound. For example, the analyzer device may be adapted to generate the ultrasound.

In general, a lysis of cells may be accomplished by using numerous techniques including mechanical (e.g. addition of glass particles, ultrasound, shearing in restricted channel), electrical (high-voltage), thermal and chemical techniques (high osmotic pressure, detergents, enzymes).

In optional step 104, additional agents may be used for binding substances released from the lysed cells. For example, non-assay magnetic particles may be added to the first chamber or cavity 26 to remove any biologically interfering substances that may be released from the cells (e.g. proteins, nucleic acids). The non-assay magnetic particles may contain binding moieties that could bind to an interfering molecule (anti-protein antibodies, charged silica surfaces for nucleic acids etc).The non-assay magnetic particles may be added during or after the lysis. A magnetic field may be used to retain the non-assay particles in the first chamber or cavity 26, when the cleaned lysate 17 is allowed to enter the chamber or cavity 30 containing the assay particles 38. This magnetic field may be generated by the analyzer device 10.

According to an embodiment of the invention, the method comprises adding a binding agent, for example non-assay magnetic particles, to the lysate 17 for binding a substance released from the lysed cells.

In optional step 106, the valve 34 opens to release the lysate to the second cavity 30. The valve 34 may be a permanent microfluidic valve (magnetically, electrically, fluidically actuated to release) or a semi permanent value. The valve 34 may be a hydrophobic valve, which comprises a hydrophobic passive structure, that, for example, is injected moulded directly in the plastic cartridge 14. The hydrophobic valve 34 may become slowly hydrophilic as proteins from the body fluid 16 adsorb to the structure. After a fixed, specific amount of time, the contact angle of the structure is such that body fluid 16 (or lysate 17) is allowed to pass. This time may be of the order of one to several minutes. The specific time may be tuned by the addition or removal of bulk proteins from the sample 16 and by adjusting the structure. The specific time may be choosen such that all cells are lysed in the first cavity 26 before the lysate 17 is released to the second cavity 30.

In general, the first cavity 26 and the second cavity 30 are separated from each other for a specific time, for example below one minute, after body fluid 16 has entered the first cavity 26.

According to the invention, the lysate 17 is mixed with the particles 38 after a specific time after the start of the lysis of the cells of the body fluid 16, and/or the substrate 38 may be exposed to the lysate 17 after the specific time.

In step 108, the lysate 17 is conveyed from the first cavity 26 to the second cavity 30 via the channel 28, for example by capillary transportation. The body fluid sample 16 may be first lysed in a separate cavity or chamber 26 and then the lysate is transported to a second cavity or chamber 30 in which the magnetic particles 38 are present. The assay may be performed in the second chamber 30 or in a subsequent chamber. The cells may be lysed before exposing them to the magnetic particles 38 as they bind to the cells, which may result in irreversible aggregation of particles 38.

According to the invention, the method comprises mixing the lysate 16 with particles 36 with first detector molecules.

According to the invention, the method comprises exposing a substrate 38 with second detector molecules to the lysate 17.

According to the invention, the cells of the body fluid 16 are lysed in the first cavity 26, and the lysate 17 is mixed with the particles 36 and/or the substrate 38 is exposed to the lysate 17 in the second cavity 30, different from the first cavity 26.

Alternatively, the lysate 17 may be generated and analyzed at the same place, for example in a single cavity or chamber of the cartridge 14.

In step 110, the magnetic particles 36 in the second cavity and the substrate 38 binds to target molecules in the lysate 17. The magnetic particles 36 may be seen as labels for the specific target molecules.

According to an embodiment of the invention, the method comprises binding target molecules to the particles 36 through first detector molecules that may be attached to the particles 36.

According to the invention, the method comprises binding target molecules to the substrate 38 through second detector molecules that may be attached to the substrate 38.

The binding may be facilitated by intermediate binding molecules (for example Streptavidin, biotin) forming a chain that links the particles 36 to the substrate surface 38 via a target molecule.

The magnetic particles may be moved by magnetic forces as described in the above.

According to an embodiment of the invention, the method comprises moving the magnetic particles 36 in direction to the substrate 38 by a magnetic force, which may be generated by the bottom magnet 42.

According to an embodiment of the invention, the method comprises removing magnetic particles 36 not attached to the substrate 38 by an opposite magnetic force, which may be generated by the top magnet 40.

In step 112, the amount of target molecules in the lysate 17 is detected as described above.

According to the invention, the method comprises detecting particles 36, for example magnetic particles 36, attached to the substrate 36.

According to an embodiment of the invention, the method comprises detecting the magnetic particles 36 attached to the substrate 38 by detecting radiation reflected by the particles.

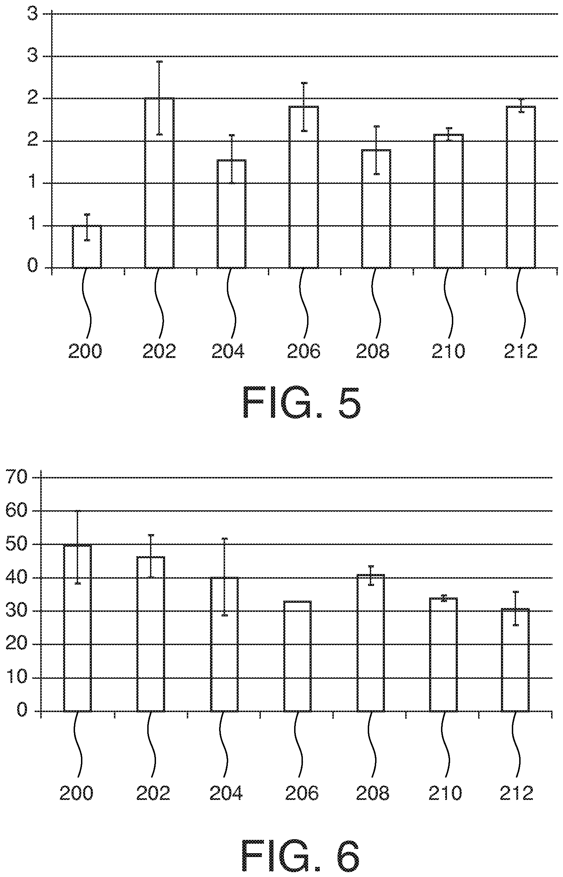

Fig. 5 shows a diagram with detection signal changes caused by lysing a human blood sample without target molecules and Fig. 6 shows a diagram with detection signal changes caused by lysing a human blood sample 16 containing target molecules in the form of cTnI.

The vertical axis of the diagrams shows the signal change of the detector 46 in percent with respect to a reference signal, that is generated, when the second cavity 30 is filled with a reference fluid.

The different bars show the detected signal for differently treated blood 16. The first bar 200 is a signal for a plasma from centrifuged blood 16 and may be used as a reference. The second bar 202 is the signal for blood 16 lysed by sonication. The other bars 204, 206, 208, 210, 212 are the signals for full blood lysed with the detergents Nonidet-P40, Nonidet-P40 substitute, sucrose monolaurate, Chemal LA-9 and Triton X100 (1% in 1x Phosphate Buffered Saline, pH 7.4), respectively.

In the case 202, the blood was sonicated for 5 seconds at 40% amplitude using a 2 mm probe. In the cases 204 to 212, the detergent was added to the blood in a 1:1 ratio and incubated for 1 minute at room temperature.

For generating the signals 202 to 212, the disposable cartridge 14 contained dry 500 nm magnetic particles coated with tracer anti-cTnI antibodies and a dry buffer consisting of HEPES, sucrose, BSA, KBr and KCl in the second cavity 30. The cTnI assay was performed using a magnetic actuation protocol consisting of 1 minute of incubation of the magnetic particles 36 with the lysate 17 and 4 minutes of pulsed actuation of the magnetic particles 36 at the substrate surface 38, on which capture anti-cTnI antibodies are coupled. Magnetic particles 38 bound to the surface 38 were detected using frustrated total internal reflection. The signals given are that after non bound magnetic particles 36 have been removed by a magnetic force away from the substrate surface 38.

As may be seen from the bars from Fig. 5 and 6, after lysis of blood by sonication or with the detergents Nonidet-P40, Nonidet-P40 substitute, sucrose monolaurate, Chemal LA-9 and Triton X100, the cTnI assay is still functional.

Summarized, by first lysing the cells in a body fluid sample 16, for example a blood sample 16, the assay with magnetic particles 36 may be performed directly in the lysed sample 17 with a low level of interference. The lysate 17 may be directly used to determine the target molecule concentration using an immunoassay with magnetic particles 36. In such a way, no analyte may be lost and no or only minor interference of cells with magnetic particles 36 may occur. A FTIR technique, which may be only sensitive to index of refraction changes and insensitive to the color, fluorescence etc., may be used for a direct detection. A steric hindrance of large cells may be avoided and a binding of magnetic particles 36 to cells may not be present.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A cartridge (14) for insertion into an analyzer device (10), the cartridge (14) comprising:
a detergent for lysing cells in a body fluid (16);
particles (36) with first specific binding molecules;
a substrate (38) with second specific binding molecules;
a first cavity (26) and a second cavity (30);
wherein the detergent is arranged in the first cavity (26);
wherein the particles are arranged in the second cavity (30).
wherein the first cavity (26) and the second cavity (30) are connected by a channel (28);
wherein the substrate is arranged in the second cavity (30),
the cartridge (14) further comprising:
a valve (34) in the channel (28);
wherein the valve (34) is adapted to open after a specific time after the body fluid (16) has entered the first cavity (26).

2. A method of determining a presence of target molecules in a body fluid (16) comprising cells, the method comprising the steps of:
lysing cells in the body fluid (16) for generating a lysate (17);
mixing the lysate (16) with particles (36) with first specific binding molecules;
binding target molecules to the particles through first specific binding molecules;
exposing a substrate (38) with second specific binding molecules to the lysate (17);
binding target molecules to the substrate through second specific binding molecules;
detecting particles (36) attached to the substrate (36);
wherein the cells of the body fluid (16) are lysed in the first cavity (26) of the cartridge (14) of claim 1;
wherein the lysate (17) is mixed with the particles (38) and/or the substrate (38) is exposed to the lysate (17) after a specific time after the start of the lysis of the cells of the body fluid (16) by opening the valve (34) of the cartridge (14);
wherein the lysate is mixed with the particles (36) in the second cavity (30) of the cartridge (14).

3. The method of claim 2,
wherein the cells are lysed by a detergent or a mixture of detergents.

4. The method of claim 3,
wherein the detergent comprises at least one of Nonidet-P40, sucrose monolaurate, Chemal LA-9 and Triton X100.

5. The method of claim 2 or 3,
wherein at least a salt is added to the detergent in absence of proteins and carbohydrates.

6. The method of one of claims 2 to 5,
wherein the cells are further lysed by a physical lysing method.

7. The method of one of claims 2 to 6,
wherein the substrate (38) is exposed to the lysate in the second cavity (30).

8. The method of one of claims 2 to 7, further comprising the step of:
adding a binding agent to the lysate (17) for binding a substance released from the lysed cells.

9. The method of one of claims 2 to 8,
wherein the target molecules are a specific type of protein.

10. The method of one of claims 2 to 9,
wherein the body fluid (16) comprises blood.

11. The method of one of claims 2 to 10,
wherein the particles are magnetic particles (36);
wherein the method comprises the steps of:
moving the magnetic particles (36) in direction to the substrate (38) by a magnetic force;
removing magnetic particles (36) not attached to the substrate (38) by an opposite magnetic force;
detecting the magnetic particles (36) attached to the substrate (38) by detecting radiation reflected by the particles.

## Patentansprüche

1. Kartusche (14) zum Einführen in eine Analysevorrichtung (10), wobei die Kartusche (14) umfasst:
ein Detergens zur Lyse von Zellen in einer Körperflüssigkeit (16);
Partikel (36) mit ersten bestimmten Bindungsmolekülen;
ein Substrat (38) mit zweiten bestimmten Bindungsmolekülen;
eine erste Vertiefung (26) und eine zweite Vertiefung (30);
wobei das Detergens in der ersten Vertiefung (26) angeordnet ist;
wobei die Partikel in der zweiten Vertiefung (30) angeordnet sind.
wobei die erste Vertiefung (26) und die zweite Vertiefung (30) durch einen Kanal (28) verbunden sind;
wobei das Substrat in der zweiten Vertiefung (30) angeordnet ist,
wobei die Kartusche (14) weiter umfasst:
ein Ventil (34) in dem Kanal (28);
wobei das Ventil (34) ausgeführt ist, um sich nach einer bestimmten Zeit zu öffnen, nachdem die Körperflüssigkeit (16) in die erste Vertiefung (26) eingetreten ist.

2. Verfahren zur Bestimmung einer Präsenz von Zielmolekülen in einer zellenenthaltenden Körperflüssigkeit (16), wobei das Verfahren die Schritte umfasst:
die Lyse von Zellen in der Körperflüssigkeit (16) zum Erzeugen eines Lysats (17);
Mischen des Lysats (16) mit Partikeln (36) mit ersten bestimmten Bindungsmo lekülen;
Binden von Zielmolekülen mit den Partikeln durch erste bestimmte Bindungsmo leküle;
Aussetzen eines Substrats (38) mit zweiten bestimmten Bindungsmolekülen dem Lysat (17);
Binden der Zielmoleküle mit dem Substrat durch zweite bestimmte Bindungsmo leküle;
Erfassen von Partikeln (36), die am Substrat (36) angebracht sind;
wobei die Zellen der Körperflüssigkeit (16) in der ersten Vertiefung (26) der Kartusche (14) nach Anspruch 1 lysiert werden;
wobei das Lysat (17) mit den Partikeln (38) gemischt wird und/ oder das Substrat (38) dem Lysat (17) nach einer bestimmten Zeit nach dem Start der Lyse der Zellen der Körperflüssigkeit (16) durch Öffnen des Ventils (34) der Kartusche (14) ausgesetzt wird;
wobei das Lysat mit den Partikeln (36) in der zweiten Vertiefung (30) der Kartusche (14) gemischt wird.

3. Verfahren nach Anspruch 2,
wobei die Zellen durch ein Detergens oder ein Gemisch von Detergenzien lysiert werden.

4. Verfahren nach Anspruch 3,
wobei das Detergens zumindest eines aus Nonidet-P40, Saccharosemonolaurat, Chemal LA-9 und Triton X100 umfasst.

5. Verfahren nach Anspruch 2 oder 3,
wobei dem Detergens zumindest ein Salz in Abwesenheit von Proteinen und Kohlenhydraten beigegeben wird.

6. Verfahren nach einem der Ansprüche 2 bis 5,
wobei die Zellen weiter durch eine physikalisches Lyseverfahren lysiert werden.

7. Verfahren nach einem der Ansprüche 2 bis 6,
wobei das Substrat (38) dem Lysat in der zweiten Vertiefung (30) ausgesetzt wird.

8. Verfahren nach einem der Ansprüche 2 bis 7, weiter den Schritt umfassend:
Beigeben eines Bindemittels zu dem Lysat (17) zum Binden einer Substanz, die aus den lysierten Zellen gelöst wird.

9. Verfahren nach einem der Ansprüche 2 bis 8,
wobei die Zielmoleküle eine bestimmte Art von Protein sind.

10. Verfahren nach einem der Ansprüche 2 bis 9,
wobei die Körperflüssigkeit (16) Blut umfasst.

11. Verfahren nach einem der Ansprüche 2 bis 10,
wobei die Partikel magnetische Partikel (36) sind;
wobei das Verfahren die Schritte umfasst:
Bewegen der magnetischen Partikel (36) durch eine Magnetkraft in Richtung des Substrats (38);
Entfernen der nicht auf dem Substrat (38) angebrachten magnetischen Partikel (36) durch eine entgegengesetzte Magnetkraft;
Erfassen der auf dem Substrat (38) angebrachten magnetischen Partikel (36) durch Erfassen von Strahlung, die durch die Partikel reflektiert wird.

## Revendications

1. Cartouche (14) pour insertion dans un dispositif d'analyse (10), la cartouche (14) comprenant :
un détergent pour lyser des cellules dans un fluide corporel (16) ;
des particules (36) avec des premières molécules de liaison spécifiques ;
un substrat (38) avec des deuxièmes molécules de liaison spécifiques ;
une première cavité (26) et une deuxième cavité (30) ;
dans laquelle le détergent est agencé dans la première cavité (26) ;
dans laquelle les particules sont agencées dans la deuxième cavité (30) ;
dans laquelle la première cavité (26) et la deuxième cavité (30) sont reliées par un canal (28) ;
dans laquelle le substrat est agencé dans la deuxième cavité (30),
la cartouche (14) comprenant en outre :
une soupape (34) dans le canal (28) ;
dans laquelle la soupape (34) est adaptée pour s'ouvrir après un temps spécifique après que le fluide corporel (16) est entré dans la première cavité (26).

2. Procédé de détermination d'une présence de molécules cibles dans un fluide corporel (16) comprenant des cellules, le procédé comprenant les étapes consistant à :
lyser des cellules dans le fluide corporel (16) pour générer un lysat (17) ;
mélanger le lysat (16) avec des particules (36) avec des premières molécules de liaison spécifiques ;
lier des molécules cibles aux particules par le biais des premières molécules de liaison spécifiques ;
exposer un substrat (38) avec des deuxièmes molécules de liaison spécifiques au lysat (17) ;
lier des molécules cibles au substrat par le biais des deuxièmes molécules de liaison spécifiques ;
détecter des particules (36) attachées au substrat (36) ;
dans lequel les cellules du fluide corporel (16) sont lysées dans la première cavité (26) de la cartouche (14) selon la revendication 1 ;
dans lequel le lysat (17) est mélangé avec les particules (38) et/ou le substrat (38) est exposé au lysat (17) après un temps spécifique après le début de la lyse des cellules du fluide corporel (16) en ouvrant la soupape (34) de la cartouche (14) ;
dans lequel le lysat est mélangé avec les particules (36) dans la deuxième cavité (30) de la cartouche (14).

3. Procédé selon la revendication 2,
dans lequel les cellules sont lysées par un détergent ou un mélange de détergents.

4. Procédé selon la revendication 3,
dans lequel le détergent comprend au moins l'un parmi du Nonidet-P40, du monolaurate de sucrose, du Chemal LA-9 et du Triton X100.

5. Procédé selon la revendication 2 ou 3,
dans lequel au moins un sel est ajouté au détergent en l'absence de protéines et de carbohydrates.

6. Procédé selon l'une des revendications 2 à 5,
dans lequel les cellules sont en outre lysées par un procédé de lyse physique.

7. Procédé selon l'une des revendications 2 à 6,
dans lequel le substrat (38) est exposé au lysat dans la deuxième cavité (30).

8. Procédé selon l'une des revendications 2 à 7, comprenant en outre l'étape consistant à :
ajouter un agent de liaison au lysat (17) pour lier une substance libérée des cellules lysées.

9. Procédé selon l'une des revendications 2 à 8,
dans lequel les molécules cibles sont un type spécifique de protéine.

10. Procédé selon l'une des revendications 2 à 9,
dans lequel le fluide corporel (16) comprend du sang.

11. Procédé selon l'une des revendications 2 à 10,
dans lequel les particules sont des particules magnétiques (36) ;
dans lequel le procédé comprend les étapes consistant à :
déplacer les particules magnétiques (36) en direction du substrat (38) par une force magnétique ;
retirer les particules magnétiques (36) non attachées au substrat (38) par une force magnétique opposée ;
détecter les particules magnétiques (36) attachées au substrat (38) en détectant un rayonnement réfléchi par les particules.
